# EUROPEAN PATENT APPLICATION

(11) **EP 1 629 807 A1**
(43) Date of publication of application: **01.03.2006**
(21) Application number: 04020177.4
(22) Date of filing: 25.08.2004
(51) Int. Cl.: A61F 11/08

(54) **Hearing protection earplug, method for manufacturing the same and method for detecting an earplug**

(71) Applicant: PHONAK AG, 8712 Stäfa (CH)
(72) Inventor: Haussmann, Mathias, 8005 Zürich (CH); Berg, Christian, 8713 Uerikon (CH)
(74) Representative: Schwan - Schwan - Schorer

(57) **Abstract**

The invention relates to a passive hearing protection earplug for being worn at least partly within the ear canal of a person, comprising a hard shell (1000) with an elasticity of from shore D85 to shore D65 having an outer surface individually shaped according to the measured inner shape of the person's outer ear and ear canal and being adapted to attenuate sound waves arriving at the outer end of the earplug, further comprising tracing means (1002, 1004, 1006, 1010, 1013, 1014) adapted for being detected by an electromagnetic detector, said tracing means being integrated within or fixed at said shell. The invention also relates to a method for manufacturing and a method for detecting such an earplug.

## Description

The present invention relates to a customized passive hearing protection earplug, a method for manufacturing such an earplug and a method for detecting an earplug when lost.

A large part of the population is exposed to hazardous noise from time to time. This can be at work, whilst traveling, during leisure activities or at home. The exposure can lead to permanent hearing loss, distract people's attention from other hazards or simply cause stress. In order to prevent both accidents and permanent hearing damage, hearing protection devices (HPDs) have been provided in many styles and over many years. It started with the earmuff which is still very relevant and addresses very noisy environments (e.g. airports, construction, shooting) or complex working/communication situations (e.g. fighter pilots). Over the years development of biocompatible soft materials has enabled soft earplugs in different styles and colors as well as recent development of "one fits many" standard semi-soft earplugs in silicon-rubber type materials. For severe situations even the combination of an earmuff and an "in-the-ear" HPD is required to achieve desired attenuation. The physical limitation of hearing protection based on ear worn devices is defined where bone-conduction (body acoustics) becomes dominant at around 40dB attenuation.

A common disadvantage of the above mentioned HPD styles is wearing discomfort. In case of the earmuffs, they are large which creates difficulties in combination with other head worn gear and they "close off" the ear too much for most applications. The in-the-ear styles mentioned are devices made to fit "the average" ear in one way or the other. Either the fit is provided by softness of the material which leads to undefined device insertion and undefined attenuation, or the fit is provided by standard shaped structures intended to block off the ear canal. In both cases the flat distribution of the individual shape of the outer ear and the ear canal leads to bad fit, pressure points in the ear and undefined positioning of the device.

To address this wearing comfort issue, in-the-ear hearing aid technology has been applied making customized ear molds with passive acoustical filter. These are long lasting devices with good wearing comfort. However, this customization process is traditionally a very manual process creating varying results over time, low reproducibility and the quality is very operator skill dependent.

Customized earplugs are earplugs comprising a hard shell which has an outer surface individually shaped according to the measured inner shape of the user's outer ear and ear canal. Such earplugs presently are primarily used for housing hearing aids. The inner shape of the user's outer ear and ear canal may be measured, for example, by direct laser scanning or by forming an impression. The customized hard shell may be produced by an additive process, such as layer-by-layer laser sintering of a powder material. Customized earplugs are described, for example, in WO 02/071794 A1, US 2003/0133583 A1 or US 6,533,062 B1.

On the other hand, soft earplugs are widely used, in particular also as hearing protection devices. A soft earplug has an outer surface with a standardized shape is made of a relatively soft material so that the outer surface of the earplug is capable of adapting its shape to the individual inner shape of the user's outer ear and ear canal.

Hearing protection devices are primarily used in industrial environments where production machines produce noise levels requiring protection. In certain industries special measures must be taken to maintain hygienic standards. In the food processing industry such measures will include detection of any foreign substances or objects in the product so that clean room or close to clean room standards apply. When hearing protection devices are used in the food processing industry, this requirement implies that the hearing protection devices must remain detectable when lost into any substance, in particular also when no longer visible.

EP 0 580 704 B1 and EP 1 006 968 B1 relate to soft hearing protection earplugs which have a metal powder homogeneously disbursed within the earplug material in order to make the earplug electrically or magnetically detectable when used in the food processing industry.

For the same purpose, EP 0 386 220 B1 and WO 00/40188 describe the application of a metal ball insert in soft hearing protection earplugs.

According to EP 0 244 979 B1 a metallic ferrule is provided at each end of a cord connecting two soft hearing protection earplugs for making this couple of earplugs detectable when used in the food processing industry.

EP 0 768 241 B1 relates to a soft hearing protection earplug which is provided with a metal band around the periphery of a part of the earplug for making the earplug detectable when used in the food processing industry.

It is an object of the present invention to provide for a passive hearing protection earplug which has optimized fit within the user's ear canal and which should be easily detectable when being lost and no longer visible. Further objects are to provide for an efficient method for manufacturing such an earplug and to provide for a method for detecting such an earplug when lost.

These objects are achieved by a hearing protection earplug as defined in claim 1, manufacturing methods as defined in claims 27 and 28 and methods for detecting such earplugs when lost as defined in claims 35 and 36, respectively.

The invention is beneficial in that, by providing a passive hearing protection earplug with a customized hard shell, with tracing means adapted for being detected by an electromagnetic detector being integrated in or fixed at the shell, an optimized fit within the user's ear canal and hence an optimized hearing protection function can be achieved, while at the same time the earplug can be detected if lost and no longer visible. By integrating the tracing means within the shell or by fixing the tracing means after production of the shell at the shell, such a customized hearing protection earplug can be manufactured in an efficient manner. In case that the hearing protection earplug is an active device comprising electronic components, such an earplug can be particularly easily detected by simply detecting the position of at least one of the electrical components by an electromagnetic detector.

Preferred embodiments of the invention are defined in the subclaims.

In the following, preferred examples of the invention will be illustrated by referring to the attached drawings.
- Figs. 1a and 1b: show a schematic longitudinal sectional view of one embodiment of an earplug according to the invention before and after final assembly;
- Fig. 2: shows a schematic view of the manufacturing process of an earplug according to a different embodiment;
- Figs. 3a and 3b: show views like Fig. 1a and 1b, with another embodiment being shown;
- Fig. 4: shows a longitudinal sectional view of the face plate of an earplug in a first use mode;
- Fig. 5: shows a view like Fig. 4, with a second use mode being shown;
- Fig. 6: shows a view like Fig. 4, with a third use mode being shown;
- Fig. 7: shows a longitudinal sectional view of the filter portion of an earplug;
- Fig. 8: shows a top view of an acoustic filter to be used in the earplug of Fig. 7;
- Figs. 9 and 10: show a longitudinal sectional view of an earplug comprising a communication button in two different functional positions;
- Fig. 11: shows a schematic longitudinal sectional view of a portion of a modified embodiment of an earplug with a communication button;
- Fig. 12: shows a view like Fig. 1b, with a modified embodiment being shown;
- Fig. 13: shows a schematic longitudinal sectional view of an another example of a passive hearing protection earplug according to the invention; and
- Fig. 14: shows a schematic longitudinal sectional view of an example of an hearing protection earplug to be used with the invention.

The present invention generally relates to customized passive hearing protection earplugs comprising a hollow hard shell which has an outer surface individually shaped according to the measured shape of the user's outer ear and ear canal. The hard shell preferably has an elasticity of from shore D85 to shore D65 and preferably is made of nylon. In order to achieve an individually customized shape of the outer surface of the hard shell, prior to manufacturing the individual shape of the ear canal has to be determined, for example, by direct three-dimensional laser scanning of the ear canal and the concha or by producing an impression of the ear canal and the concha. In the first case, digital data of the shape of the ear canal and the concha is directly determined, while in the latter case such data is obtained from the impression, e.g. by three-dimensional scanning of the impression.

The digital data obtained thereby then may be used to create the hard shell by an additive build-up process. Such additive or incremental building processes are also known as "rapid prototyping". An overview regarding such processes can be found, for example, in US 2003/0133583 A1 or US 6,533,062 B1.

A preferred incremental additive build-up process is a layer-by-layer laser sintering process of powder material. Such processes are also defined as "selective laser sintering" (SLS). The basic principle in the incremental build-up process consists in the repeated deposition of a thin layer of material on the surface, with the desired sectional shape then being stabilized, i.e. hardened, by laser action.

A preferred process is available under the trademark Nemotech from applicant, wherein an impression of the ear canal is taken and then undergoes three-dimensional laser scanning with high resolution. The thus obtained three-dimensional data undergo shell modelling for determining the best possible fit. Then the hard shell is produced by selective laser sintering from polyamide powder. Subsequently the outer surface of the hard shell is smoothed and provided with a skin-like texture to ensure a firm fit and good retention in the ear canal.

In general, a hearing protection earplug is adapted to attenuate sound waves arriving at the outer end of the earplug when the earplug is worn at least partially within the ear canal of the user. For this purpose, the earplug, i.e. the hard shell, may comprise a passive acoustic filter element which is adapted to attenuate sound transmission through the earplug. Further, the earplug, i.e. the hard shell, may comprise an outer opening for sound input at its outer end from which sound passes to the acoustic filter element. Alternatively, the acoustic filter(s) may be provided at an outer faceplate closing the shell at its outer end.

Passive hearing protection earplugs usually do not include any electronic components.

According to the present invention, the hard shell in general comprises tracing means adapted for being detected by an electromagnetic detector so that the earplug can be detected by an electromagnetic detector such as an X-ray detector, a metal detector or a magnetic detector, if it has been lost and is no longer visible. This is particularly important for earplugs being used in the food processing industry, wherein any contamination of process substances by foreign substances or articles is to be strictly avoided. Such tracing means may be integrated within the shell during manufacturing of the shell or may be fixed at the shell after manufacturing of the shell. Preferably, the tracing means comprises metal.

Currently, for example, in the food processing, tobacco processing, beverage producing and the pharmaceutical industries electromagnetic detectors such as X-ray detectors, a metal detectors, a magnetic detectors or similar scanning devices are used for detecting the presence and position of foreign objects in the processed product.

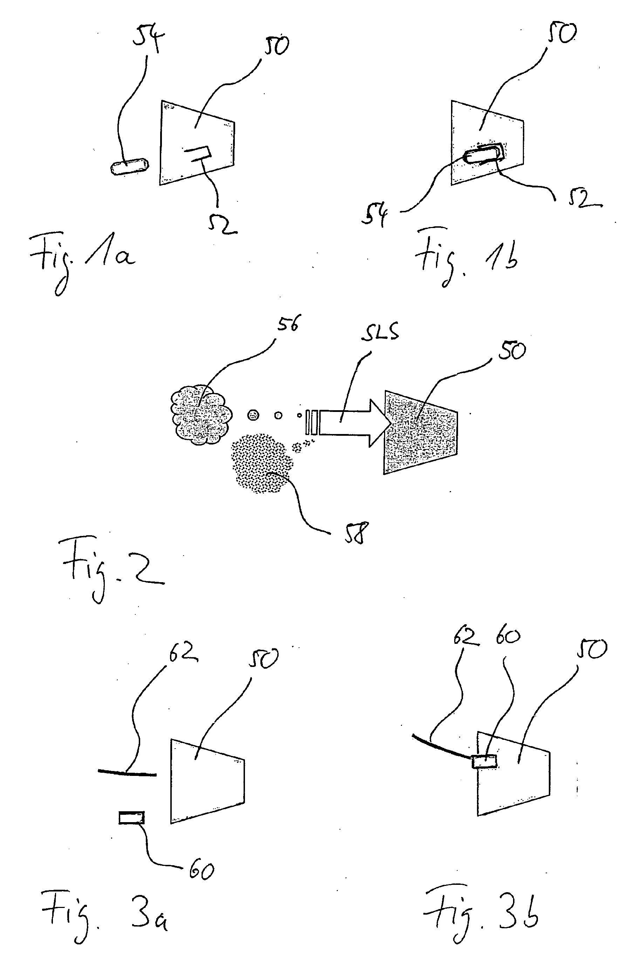

Figs. 1a and 1b show schematically an example, wherein a customized hard shell 50 of a hearing protection earplug is manufactured with a pre-shaped receptacle portion 52 which is adapted for receiving a metal part 54 as the tracing means; i.e. the receptacle portion 52 is shaped during the incremental build-up process of the hard shell 50. Subsequently the metal part 54 is inserted into the receptacle portion 52 for being fixedly held there, see Fig. 1b. To this end, the metal part 54 may be pressure mounted during earplug assembly in the receptacle portion 52.

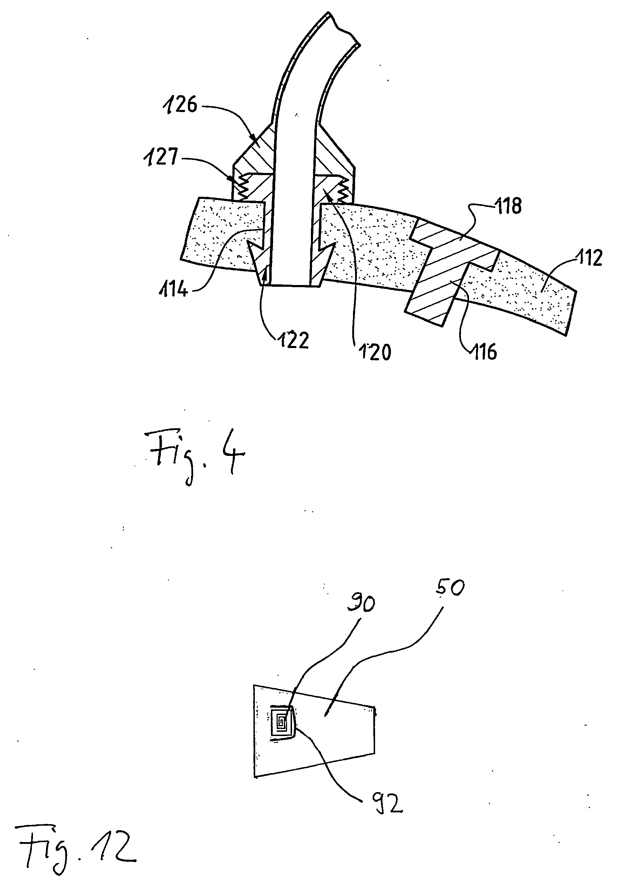

Fig. 2 is a schematic representation of an alternative embodiment, wherein the tracing means is formed by metal particles 58 dispersed, preferably homogeneously, within the material of the hard shell 50. This may be accomplished by mixing, prior to manufacturing of the shell 50, the shell powder material 56 with the metal particles 58. Subsequently, the mixed powder is used for selective laser sintering of the hard shell 50, whereby a homogeneous distribution of the metal particles 58 within the shell 50 is achieved. Preferably, the metal particles 58 may have an average particle size from 5 to 100 µm.

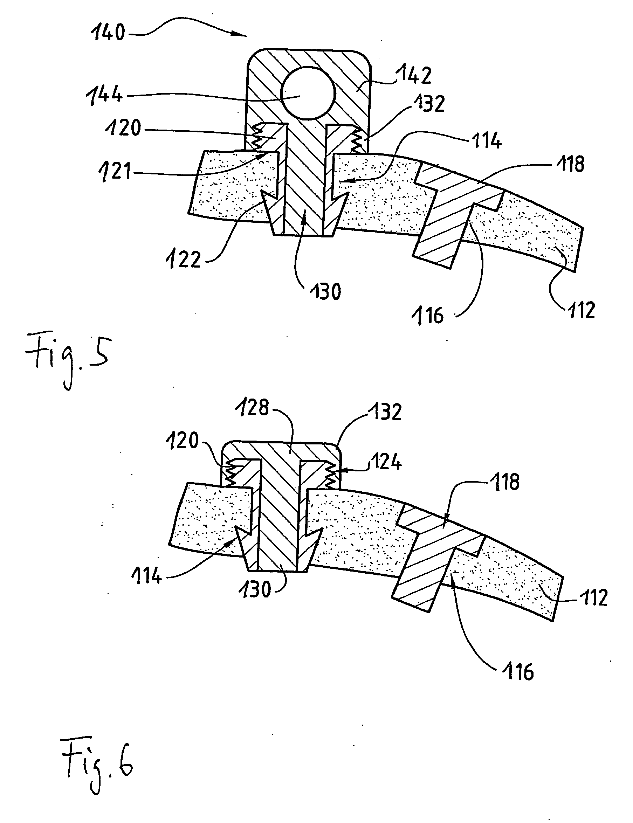

In Figs. 3a and 3b an example is schematically shown wherein the tracing means is a functional element for providing at least one additional function (i.e. at least one function in addition to the tracing function) to the earplug. In particular, the embodiment of Figs. 3a and 3b is an example of the case in which the functional element is at least part of a support element. More specifically, Figs. 3a and 3b show a support element 60 which is to be mounted at the outer end of the hard shell 50 after manufacturing of the hard shell 50 and which serves to fix an external part 62, such as an external security cord, to the earplug.

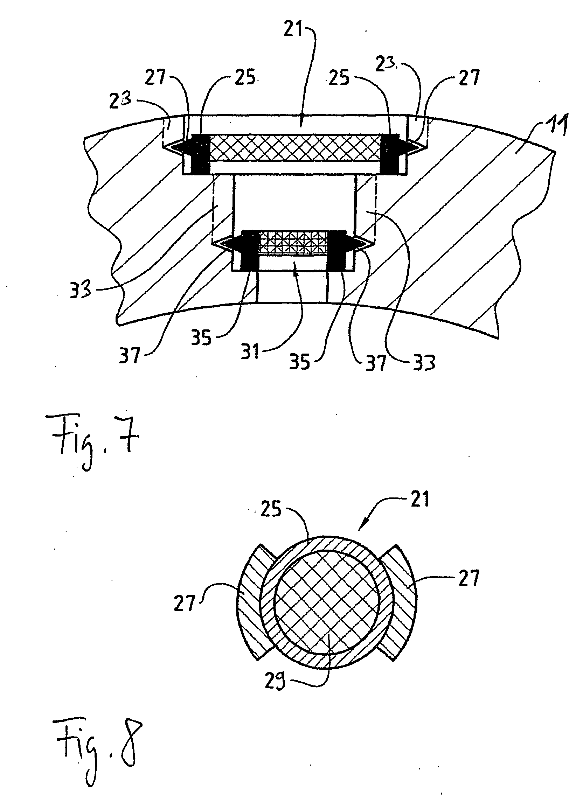

A more detailed example of the general concept illustrated in Fig. 3a and 3b is shown in Fig. 4 to 6. Fig. 4 is a vertical cross-sectional view of the outer portion of a hearing protection earplug, comprising faceplate 112 which has two holes 114, 116. The holes 114, 116 may typically be circular. However, by using for example laser sintering techniques it is easy to provide also other shapes for the hole 114, 116. The hole 116 is connected to a duct leading into the ear whereas hole 114 is provided for being temporarily connected with a flexible tube for attenuation measurements.

In a manner known per se, the hole 116 is closed by an acoustic device 118 that is the attenuating element in that it generally comprises an acoustic filter for noise frequency response modification.

A fitting element 122 in the form of a hollow, generally cylindrical sleeve, is inserted into and fixed in hole 114 normally in a permanent manner, typically by cementing. However, it can also be provided in a removable manner or as a part of the faceplate 112. The fitting element 122 has an outer annular circular flange 120 whose outer diameter is greater than that of the fitting body 122. The outer periphery of the flange 120 can e.g. be provided with a male thread 124.

As it can be seen in Fig. 4, a connector 126 of a sound measuring tube has an inner female thread in its hollow cylindrical front portion. This thread is screwed upon the male thread 124 of the fitting flange 120 so that the measuring tube is firmly but in a removable manner connected to the faceplate and thus to the interior of the hearing protection earplug.

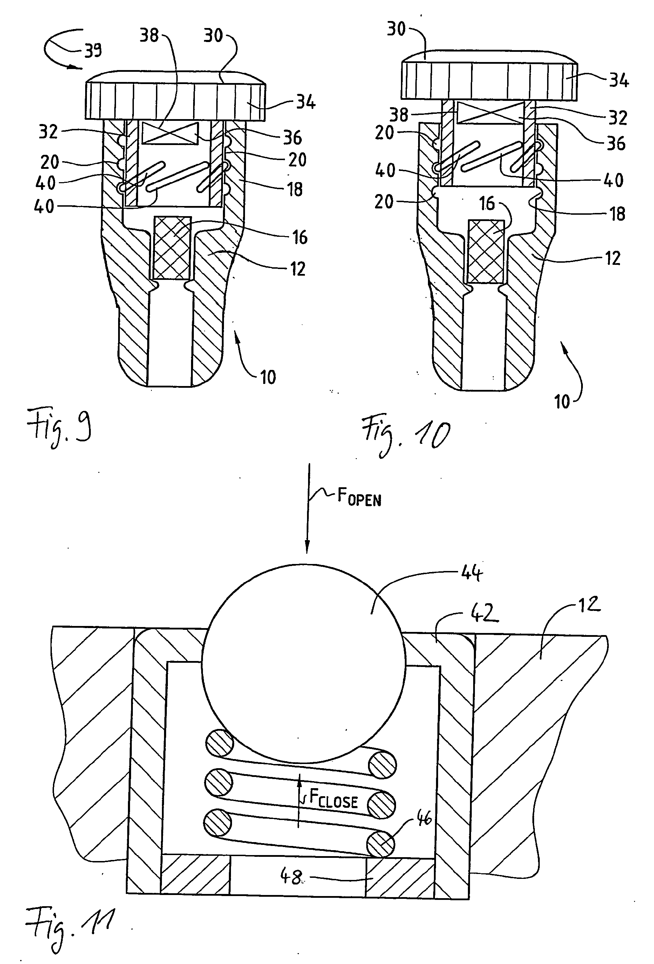

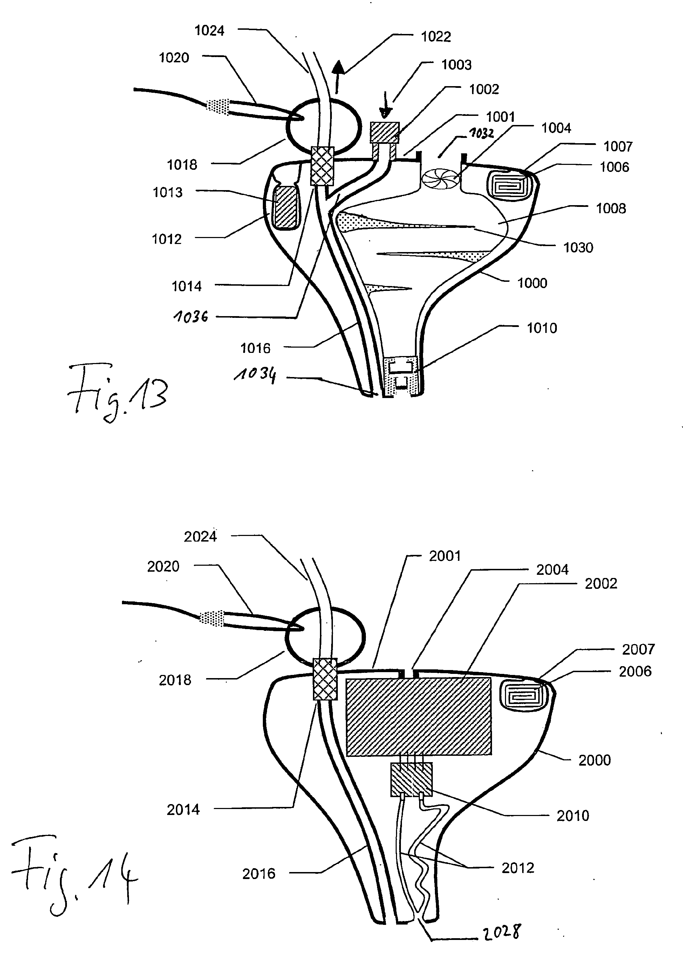

When the measurement is finished, the tube connector 126 is screwed off, and the device is secured in that a screwable security plug 128 is screwed on the thread 124 of the fitting 122, see Fig. 6. The security plug 128 comprises a cylindrical stem 130 filling the interior of the fitting element 122. The plug 130 further comprises an outer rim 132 bearing a female thread 127 on its inner peripheral surface so that the plug 128 can closely screwed in the manner of a cap onto the fitting element 122.

The security plug 128 may comprise means for wireless communication to a wireless communication network or any other electronic communication device.

When the hearing protection earplug is to be used and to be connected to a cord, the plug 128 is unscrewed and replaced by an adapter plug 140 which is screwed on the thread 124 of the fitting element 122, see Fig. 5. This plug 140 is similar to plug 128 but is prolonged outwardly by a flat cord fastening tab 142. A cord fastening eyelet 144 is cut out of the flat tab 142. A security cord (not shown) that prevents the hearing protection earplug from being lost can now be fastened in this eye in any desired manner.

The flat cord fastening tab may be replaced by a cylindrical stem protruding from the plug and having a transverse bore for attaching the security cord. This embodiment is not shown.

The flat tab 142 that also serves as a handle for an easy application and removal of the combined plug 140 may be replaced by any other fastening element, e.g. a simple half-circular loop. Furthermore, the connecting threads 124, 127 may be replaced by any other connecting means known per se.

In the embodiment shown in Figs. 4 to 6 the fitting element 122 may be made of metal, thus serving as a tracing means. Alternatively or in addition, the adapter plug 140 may be made of metal, thereby likewise serving as a tracing means. Finally, the plug 128, if designed as an active security plug, will include electronic parts which may serve as a tracing means which can be detected by an electromagnetic detector. Alternatively or in addition also the filter element 118 may comprise metal, thereby serving as a tracing means.

Figs. 7 and 8 show an example, wherein the functional element forming the tracing means is part of a passive acoustic filter element.

The hearing protection earplug of Fig. 7 comprises a shell 10 which comprises at the distal end of the earplug, or equivalently at the end of the earplug towards the faceplate, a through-hole or cavity which is adapted to the conditions of the hearing protection earplug wearer and has been determined and manufactured during the fabrication of the customized shell 10. The cavity comprises orifices of different sizes which may be ordered, for example, with respect to their diameters as shown in Fig. 7.

Passive acoustic filters 21 and 31 installed into two of the orifices. For the fixation of the acoustic filters in the orifices, any suitable technique can be applied. In Fig. 7, for example, circular grooves in the vertical walls of the orifices are shown. The walls further comprise a pair of cylindrical recesses 23 in the upper wall and another pair of cylindrical recesses 33 in the lower wall. These recesses are shown in dotted lines in Fig. 7. Each recess 23, 33 extends, for example, over about 90° of the circumference of the corresponding wall, each wall portion having two cylindrical recesses equally spaced over the circumference. These recesses serve to the insertion of the passive acoustic filters 21 and 31. One of them, for example the passive acoustic filter 21, is represented as a top view in Fig. 8.

The passive acoustic filter 21, see also Fig. 8, can have e.g. a mainly cylindrical filter body 25 made from an appropriate material which preferably is bio-compatible and/or sterilizable. The body 25 is hollow and contains a sound attenuating material 29 the structure and nature of which has been selected beforehand as a result of the user's conditions and needs. One of the issues of the material is to provide an acoustic membrane. The filter function is given by the structure (e.g. membrane) rather than the material. The mentioned attenuation is given by the overall filter, the membrane forming part of the filter and defining part of the filter characteristics (e.g. membrane gives high-pass, tube gives low-pass etc.). The combination results in the advanced acoustic filters. This sound attenuating material typically (but not exclusively) consists of a set of filter membranes (as known per se) selected and assembled according to the desired use and customized for a particular user. It may also be possible to have the user adapt the membranes to particular conditions; for this purpose, the membranes may be removable.

The upper and lower horizontal bordering surfaces of the passive acoustic filters 21, 31 may be sound transparent in a manner known per se. For example, the passive acoustic filter bodies 25, 35 have the general shape of cylinders. Thickness and/or diameter of a passive acoustic filter and therefore the filter bodies 25, 35 may vary according to the attenuation or frequency shaping needs that shall be reached or according to the orifice in which the passive acoustic filter shall be installed. For example, a pair of projections 27, 37 is attached to the bodies 25, 35 of the passive acoustic filters 21, 31, whereas the projections 27, 37 are shaped to fit corresponding grooves.

The insertion of the passive acoustic filters 21, 31 into the orifices 20, 30 is simple. The passive acoustic filter 31 is inserted first. The filter body 35 is introduced from above into the orifice 30 and rotated until the two pairs of projections are aligned with the corresponding recesses 34 respectively, and the passive acoustic filter now falls home into the orifice 30. The filter body 35 is now turned by about 90° so that the projections remain captured within the grooves 33. The insertion of the passive acoustic filter 21 is done accordingly.

Suitable means (not shown) may be provided to retain the passive acoustic filters 21, 31 in their inserted position. For example, the grooves may have a depth that diminishes in the regions where the projections 27, 37 should be blocked, or the projections may be shaped in an appropriate manner to be clamped in the grooves; this possibility is indicated in Fig. 7 where the outer generally circular contour of the projections 27 is slightly eccentrically shaped.

The passive acoustic filters 21, 31 are removed from the orifices in executing the above-indicated steps in the reverse manner.

The described arrangement of orifices and passive acoustic filters 21, 31 form a so called semi-integrated passive filter. Main parameters for adjusting the filter characteristics of the semi-integrated passive filter are number of orifices, sizes of orifices, as well as sizes and material of the passive acoustic filters. These parameters are determined when the earplug is manufactured, for example according to an acoustic model of an individual's ear-canal with inserted earplug. When manufactured, the geometry of the orifices cannot be changed easily anymore. However, according to the procedure described above, passive acoustic filters 21, 31 are easily exchangeable. Therefore, different acoustic damping needs, for example when the wearer of the earplug moves from one noisy environment into another environment with different noise characteristics, can simply be realized by an appropriate exchange of passive acoustic filters 21, 31. However, this does not work for all applications, since the membrane property is given mostly by the structure and not by the material. Dampening in contrast is also impacted by the material used. However, to achieve significant effects it has e.g. to be based on the whole cavity or a larger cavity. The simple exchange of the passive acoustic filters 21, 31 provides as well ease of service of the earplug, for example a cleaning of the shell 11 and replacement of the passive acoustic filters 21, 31 after exposure to a dirty environment. Further, it is possible to define a standard set of passive acoustic filters 21, 31 and use parameters of the orifices for a proper design of the semi-integrated passive filter. A standard set of passive acoustic filters will lead to lower manufacturing costs and lower service costs as well.

In the example shown in Figs. 7 and 8 the passive acoustic filter bodies 25 and 35, respectively, may be made of metal, thereby acting as a tracing means.

Figs. 9 to 11 show two embodiments wherein the functional element acting as a tracing means forms part of a communication button mechanism which is manually operable between an attenuation position and a communication position, wherein in the communication position the sound attenuation of the earplug is lower than in the attenuation position. In particular, the communication button mechanism comprises a button which, in the attenuation position, closes the outer end of the shell and which, in the communication position, cooperates with the shell in order to provide for a sound channel into the interior of the shell.

The hearing protection earplug 10 shown in Figs. 9 and 10 comprises a hollow customized shell 12 to be introduced into the auditory canal of an ear. An audible frequency filter 16 is optionally inserted into the central elongated channel of the shell 12.

The cylindrical inner wall of the upper, cylindrical and hollow portion 18 of the shell 12 has a number of partial turns 20 of a relatively steep female thread cut as grooves into the wall of this cylindrical portion.

A button 30 is inserted from above into the upper cylindrical portion 18 of the shell 12. The button 30 is provided with reeding so that it may better be actuated manually. The button 30 comprises a disk like top portion 34 and a downward directed, hollow cylindrical, integrally formed sleeve-like portion 32. This sleeve 32 has a rectangular triangle cut-out 36; the long leg of the triangle 36 being parallel to the upper end plane of the cylindrical portion 18 or to the lower surface of the top portion 34 of the button 30. The corner of the triangle formed by the short leg and the hypotenuse touches the lower surface of the top portion 34 of the button 30.

A second triangular cut-out 38, drawn in dotted lines, may be provided on the diametrically opposed side of the sleeve 32.

Outer ribs 40 that are inclined to the horizontal plane in Figs. 9 and 10 form a steep multiple male thread. These ribs 40 are engaged into the grooves 20 of the shell 12. The first assembly of the device is possible thanks to the resilience of the material that yields when the button 30 is forced from above into the upper portion 18 of the shell 12.

As it can be seen in comparing Figs. 9 and 10, the attenuating action of the earplug is at a maximum when it is in the position shown in Fig. 9. When the button 30 is turned counterclockwise, i.e. in the direction of arrow 39, into the open position shown in Fig. 10, the cut-out 36 is gradually opened when the button 30 rotates and simultaneously raises from its rest or closed position, and in the open (and elevated) position, the attenuation is at a minimum. The two positions of the button differ from each other by an angle of rotation of from about 40° to about 120°, preferably of about 70 to 100°, depending on the size of the cut-out 36.

In the embodiment shown in Fig. 11, an insert 42 is inserted within an opening of a customized shell 12. The insert 42 is provided with an outer opening into which a ball 44 is inserted which is forced by a spring 46 into a closed position within the outer opening of the insert 42. The spring 46 is supported by a base plate 48 having an opening towards the interior of the shell 12. By manually pressing down the ball 44 against the biasing force of the spring 46, the ball 44 may be used as a communication button.

In the embodiment shown in Figs. 9 and 10 for example the button 30 may be made of metal or may comprise metal parts, thereby acting as a tracing means. In the embodiment of Fig. 11, for example the ball 44, the insert 42 or the spring 46 may be made of metal, thereby acting as a tracing means.

Fig. 12b shows schematically an example, wherein, similar to the embodiment of Figs. 1a and 1b, a customized hard shell 50 of a hearing protection earplug is manufactured with a pre-shaped receptacle portion 92 which is adapted for receiving tracing means; i.e. the receptacle portion 92 is shaped during the incremental build-up process of the hard shell 50. However, in this embodiment the tracing means is not a metal part but rather a radio frequency identification (RFID) tag 90 which is inserted into the receptacle portion 92 for being fixedly held there, see Fig. 12b.

A RFID-tag in general is an electromagnetic identification element which is adapted to provide, upon receipt of an electromagnetic input signal from a transmitting device, an electromagnetic identification signal which is individual (i.e. in the present case individual to the earplug) and which is operated at radio frequencies. Preferable, the RFID-tag is adapted to be energized by energy included in said input signal so that it does not need its own power source. Typically, the RFID-tag comprises an antenna, an analog circuit for receiving and transmitting radio frequency signals, a digital circuit and a non-volatile data memory. Alternatively, the digital circuit and the digital memory may be replaced by an appropriate acoustic surface wave device.

A general description of RFID-systems may be found, for example, in the "RFID-Handbook", 2^{nd} edition, by Klaus Finkenzeller, Wiley & Sons Ltd., April 2003.

If an earplug with a RFID-tag 90 has been lost, it may be detected and identified by transmitting the required RF input signal and detecting the corresponding RF response signal generated by the RFID-tag 90 by appropriate transmitting/receiving devices.

An advantage of using a RFID-tag as the tracing means rather than using a metal part is that the RFID-tag enables not only detection of its position but in addition enables individual identification of the earplug.

Figs. 13 and 14 show an example of a passive hearing protection earplug and an active hearing protection earplug, respectively, wherein tracing means for being detected if the earplug is lost are combined with other features which may be advantageously implemented by manufacturing the shell of the earplug by an additive build-up process, such as layer-by-layer laser sintering.

The earplug of Fig. 13 includes a customized hard shell 1000 with a faceplate 1001 at its outer (proximal) end. The faceplate includes an outer sound input opening 1032 provided with a mechanical peak clipper 1004, a multi-purpose cord adapter element 1014 with an in-situ measuring hole for optionally connecting the measuring hole to an external measuring tube 1024 or to a plug for closing the measuring hole in the normal operation of the earplug, and a sound inlet opening which is provided with a button 1002 which is manually operable in the direction 1003 to act as an attenuation button closing the sound inlet opening or as communication button opening the sound inlet opening for sound input into a sound passage 1036 which merges at its distal end with an in-situ measuring channel or tube 1016 which is acoustically connected to the measuring hole in adapter element 1014 and which extends to an inner sound opening 1034 at the inner end of the shell 1000. The sound input opening 1032 is connected to a resonance cavity 1008 with an inner mechanical structure 1030 for frequency tuning. At the distal end of the resonance cavity 1008 a semi-integrated passive acoustic filter 1010 is provided. The tubes 1036 and 1016 are formed integral with the shell 1000. Further, also an insert cavity 1007 for a RFID (radio frequency identification device)-tag 1006 and an insert cavity 1012 for a detectable metal part 1013 are formed integral with the shell 1000. At the adapter element 1014 or at the plug for closing the measuring hole of the adapter element a cord fixation ring 1018 may be provided for fixing a neck cord 1020 at the shell 1000 for preventing loss of the earplug. The ring 1018 or the cord 1020 also may serve to manually pull the earplug in the axial direction 1022.

In addition to the metal part 1013, also the adapter element 1014, the button 1002, the peak clipper 1004 or the filter 1010 may comprise metal and hence might serve as tracing means, whereby the need for the metal part 1013 might be eliminated. Examples of the adapter element 1014 and its function have been described above in connection with Figs. 4 to 6. Examples of the filter 1010 and its function have been described above in connection with Figs. 7 and 8. Examples of the button 1002 and its function have been described above in connection with Figs. 9 to 11.

The earplug of Fig. 14 is an active hearing protection earplug and includes a customized hard shell 2000 with a faceplate 2001 at its outer end. The shell 2000 includes a cavity for an active unit 2002 which may comprises a microphone, an audio signal processing unit (e.g. an amplifier), a programming interface, a volume control, a push button and a battery. The unit 2002 produces an audio signal output for an output transducer unit 2010, comprising one or several speakers/receivers which are acoustically connected via sound output channels 2012 to a sound output opening 2028. The faceplate 2001 includes a faceplate opening 2004 which may serve for sound input to the microphone of the active unit 2002 and/or for access to the programming interface, the volume control, the push button and/or the battery of the active unit 2002. Similar to the passive HPD of Fig. 13, an internal in-situ measurement channel 2016 with an adapter element 2014 at the faceplate 2001 for temporarily connecting to an external measurement tube 2024, a cord fixation ring 2018, a neck cord 2020 and a cavity 2007 for a RFID-tag 2006 are provided.

Usually the electrical components of the active hearing protection earplug, in particular the microphone and the other components of the active unit 2002 and the output transducer 2010, will contain enough metal enabling to detect the earplug by an electromagnetic detector when lost, e.g. in foodstuff, with no additional tracing means such as metal inserts being necessary.

In the following, the features and functions of the earplugs of Figs. 13 and 14 will be explained in more detail.

The key to the following features and functions is this technology's capability to model and customize the earplug both to fit the individual shape of the ear, but also to utilize the given shape and volume for additional functionality. In some cases the processed earplug (using the mentioned rapid prototyping technology) becomes the chassis for the additional function (e.g. "RFID (radio frequency identification)", "HPD detection part", "multipurpose cord adapter") or the function is fully integrated (e.g. "intelligent HPD resonator"). The following list of functions and features indicates examples of application.

### Multipurpose cord adapter

In order to confirm acoustical performance of an HPD, an in-situ measurement tube is implemented to allow measurement of attenuation when the individual wears the device. Naturally this tube needs to be closed off during normal operation. The core element of this tube is the faceplate component referred to a multipurpose cord adapter 1014, 2014 that embodies several functions and features: fixation of external in-situ measurement probe tube 1024, 2024, one possible holder of the cord fixation ring 1018, 2018 for the neck cord 1020, 2020, holder of an ergonomic pull means (e.g. the cord fixation ring 1018, 2018) for an inverse anatomy switch, holder of a plug for closing the in-situ tube during normal operation.

If the element is made of metal it can serve as a metal component for detection purposes 1013 which in that case spares an extra insert cavity 1012. The design of the multipurpose cord adapter element 1014, 2014 is given extensive freedom (shape, material, insertion/removal concept, etc.) due to the base technology used for the faceplate portion of the earplug 1001, 2001.

### Semi-integrated passive filter

In passive HPDs acoustical filters mainly serve two purposes: firstly there is the defined amount of attenuation, secondly the filter can shape the frequency response of the attenuation in order to protect some frequencies while letting others through (e.g. block low frequency noise and let speech pass above 1 kHz). The proposed base technology enables both usages of predefined component placement geometries (e.g. cavities 1012 for metal component 1013 insertion) as well as semi-integration of functions where the material itself becomes part of the solution (e.g. insert cavities, acoustical filters). The semi-integrated passive filter 1010 is a structure of the second kind, where the tubes are made in shell material while the membranes are inserted components. Selection of membranes can be done to order and individual need, hence the component remains customizable. The filter must be considered and dimensioned together with other filter means like the customizable front chamber shaping structure (or resonance cavity) 1008, 1030 (Helmholtz resonator) and the mechanical peak clipper 1004.

### Communication/attenuation button

A core function of a passive HPD is to enable temporary audio bypass for purposes like listening to speech, alarm or other desired audio signals even though they are mixed with loud noise. This is often performed by a push/return-button opening a tube either bypassing the filter of the system or leading into the in-situ measurement probe tube 1016 on the inside of the closing plug to be connected to the adapter element 1014 when the measuring tube 1024 is removed. The integration of such a device into the faceplate 1001 overcomes many drawbacks of similar standard component solutions (e.g. complex tubing, acoustical leakage). An even more integrated solution is achieved by building the switch directly into the multipurpose cord adapter core element 1014 replacing the sealing plug. If the button is made of metal it could serve as a metal piece for the detection function, thereby eliminating the need for the separate metal part 1013.

Inverse anatomy force button

A further level of integration of the on/off switch is based on the shell technology combined with the natural anatomy of the outer ear. In addition to a defined audio "leak" via a tube 1016 through the HPD, there is the alternative of creating a temporary leak between the device and the outer ear by slightly pulling the device out of the ear. This pull can be done by the cord 1020 or directly by grip and pull on the cord ring 1018. If the shell 1000 is shaped in an appropriate manner, the ear shape is such that the device will be naturally pulled back in place when the pull is relaxed.

### Intelligent passive HPD

Inserting a device into the ear principally blocks the acoustical tube (ear canal) and destroys the natural outer ear amplification and frequency shaping (open ear gain, OEG). The open ear has a natural resonance in the frequency area of the most critical speech information, hence this loss is a real loss and not normally desired. The resonance frequency is given by the length of the tube; hence there is a need for compensation of the reduced length. This can be individually modeled and implemented with a defined acoustical front (outer) chamber 1008 and artificially stretched to a desired length by a mechanical means 1030 for resonance shaping directly integrated into the shell making process, possibly in combination with frequency shaping filter 1010 and means for maximum power limiting such as a mechanical peak clipper 1004.

### Mechanical peak clipping

Many applications for HPDs experience strong variations in noise exposure over time. The extreme example is people shooting with guns (military, hunters) where speech communication in-between the actions is strongly desired and where the sound gets very loud for a short time. In active devices such conditions have been solved with so-called "peak clippers" which are fairly easy to implement in electronics and which limit the output of the device independent of the input signal while leaving the signal undistorted for normal noise levels. For a passive device this can be realized by a pressure sensitive valve 1004 opening or blocking the audio canal at the sound inlet.

### Acoustical tubing

Analog to the intelligent passive HPD acoustical shaping, several audio signal enhancements can be pursued by means of acoustical tubing for active HPD devices. Active HPDs are systems where the incoming sound is picked up by a transducer microphone system, processed electronically and converted back to the acoustical domain by a transducer receiver (loudspeaker). Many properties and artifacts of the signal can be taken care of in the electronic domain, but some remain difficult (e.g. resonance peaks, relation direct (venting) and indirect (processed) sound) and in particular the upcoming challenge of managing wideband receivers (e.g. two-way) for high-fidelity applications. Wideband output transducers 2010 made for such applications produce multiple output signals the mixing of which becomes complex. The ability to determine the shape and length of the individual acoustic tubes 2012 and their mixing point becomes a design and modeling choice at production time. Naturally such a system can be combined with the semi-integrated passive filter mentioned earlier for further degrees of freedom.

### HPD wearing compliance

Wearing of HPDs in industrial environments obliges to regulations in most countries. Assuming that the devices have the desired protective effect when they are worn (most other topics described address this very issue), the wearing itself becomes the compliance control topic. With recent developments in miniaturized RFID (radio frequency identification devices) technology, it becomes feasible to implement such devices into a customized HPD given the shell technology described. The RFID tag 1006, 2006 is inserted into a predefined cavity 1007, 2007 and when the wearer passes through gateways equipped with RFID detection systems, the positions of the two HPDs can be obtained and the control function carried out according to whether a predefined condition regarding the detected positions is fulfilled or not (e.g. separation of the HPDs according to the width of the head and height of the HPDs according to the ear height). As mentioned, the RFIDs can also serve as HPD detection devices in food production processes.

### Basic functions

Functions that conventionally are mounted components, such as a grip handle for insertion and removal of the HPD, can easily be integrated with use of the shell technology. The product design and assembly more and more becomes a software issue and the individual product is increasingly designed to order according to the specific requirements of each customer.

## Claims

1. A passive hearing protection earplug for being worn at least partly within the ear canal of a person, comprising a hard shell (11, 12, 50, 112, 1000) with an elasticity of from shore D85 to shore D65 having an outer surface individually shaped according to the measured inner shape of the person's outer ear and ear canal and being adapted to attenuate sound waves arriving at the outer end of the earplug, further comprising tracing means (25, 30, 35, 42, 44, 46, 54, 58, 60, 90, 118, 122, 128, 140, 1002, 1004, 1006, 1010, 1013, 1014) adapted for being detected by an electromagnetic detector, said tracing means being integrated within or fixed at said shell.

2. The earplug of claim 1, wherein said tracing means (25, 30, 35, 42, 44, 46, 54, 58, 60, 118, 122, 128, 140, 1002, 1004, 1010, 1013, 1014) comprises metal.

3. The earplug of one of claims 1 and 2, wherein said shell (11, 12, 50, 112, 1000) is made of a plastic material.

4. The earplug of claim 3, wherein said shell (11, 12, 50, 112, 1000) is made of polyamide.

5. The earplug of one of the preceding claims, wherein said shell (50, 1000) comprises a pre-shaped receptacle portion (52, 1012) for inserting a metal part (54, 1013) as said tracing means in said shell.

6. The earplug of claim 5, wherein said metal part (54, 1013) and said receptacle portion (52, 1012) are adapted for being pressure mounted during earplug assembly.

7. The earplug of one of claims 1 to 4, wherein said tracing means comprises metal particles (58) dispersed within the shell material (56).

8. The earplug of claim 7, wherein said metal particles (58) are homogeneously dispersed within said shell material (56).

9. The earplug of one of claims 7 and 8, wherein said metal particles (58) have an average particle size from 5 to 100 µm.

10. The earplug of one of claims 1 to 4, wherein said tracing means is a functional element (25, 30, 35, 42, 44, 46, 60, 118, 122, 128, 140, 1002, 1010, 1014) providing at least one additional function to said earplug.

11. The earplug of claim 10, wherein said functional element is at least part of a support element (60, 122, 140, 1014).

12. The earplug of claim 11, wherein said shell (11, 12, 112, 1000) is provided with an outer faceplate (1001) at its outer end, with said faceplate comprising an opening and a tubular adapter (120, 1014) comprising a sound channel and extending through said opening as said support element.

13. The earplug of claim 12, wherein said tubular adapter (120, 1014) comprises metal.

14. The earplug of one of claims 12 and 13, wherein said tubular adapter (120, 1014) is designed to alternatively connect, in a detachable manner, to at least one of a sound measuring tube (1024), a security plug (128) for closing said sound channel of said adapter, and an adapter plug (140) for closing sound channel of said adapter and being designed for connecting to an external security cord.

15. The earplug of one of claims 1 to 4, wherein said shell (11, 12, 112, 1000) comprises an acoustic filter element (16, 21, 31, 118, 1010) ithin said shell or at its outer end for attenuating sound transmission through said earplug.

16. The earplug of claim 15, wherein said functional element (25, 35) is at least part of said filter element (21, 31, 118, 1010).

17. The earplug of claim 16, wherein said filter element (21, 31) is a tube or diaphragm filter.

18. The earplug of claim 17, wherein said filter element (21, 31) is a diaphragm filter comprising a diaphragm and an annular body (25, 35) supporting said diaphragm, wherein said annular body is made of metal as said tracing means.

19. The earplug of claim 18, wherein said diaphragm filter (21, 31) is removably inserted within an opening of said shell (11).

20. The earplug of claim 10, wherein said earplug comprises a communication button mechanism (30, 42, 44, 46, 1002) manually operable between an attenuation position and a communication position, wherein in the communication position the sound attenuation of the earplug is lower than in the attenuation position, and wherein said functional element is at least part of said communication button mechanism.

21. The earplug of claim 20, wherein said button mechanism comprises a communication button (30, 44, 1002) which, in the attenuation position, closes the outer end of said shell (12, 1000) and which, in the communication position, cooperates with said shell in order to provide for a sound channel (1036, 1016) into the interior of said shell.

22. The earplug of claim 21, wherein said communication button (30, 44, 1002) comprises metal.

23. The earplug of one of claims 21 and 22, wherein said communication button mechanism (42, 44, 46, 1002) comprises a spring element (46) for biasing said communication button mechanism towards said attenuation position, said spring element comprising metal.

24. The earplug of one of claims 1 to 4, wherein said tracing means is an electromagnetic identification means (90, 1006) which is adapted to provide, upon receipt of an electromagnetic input signal, an electromagnetic identification signal which is individual to said earplug.

25. The earplug of claim 24, wherein said identification means (90, 1006) is adapted to be energized by energy included in said input signal.

26. The earplug of claim 25, wherein said identification means (90, 1006) comprises an antenna, an analog circuit for receiving and transmitting radio frequency signals, a digital circuit and a non-volatile data memory.

27. A method for manufacturing a passive hearing protection earplug for being worn at least partly within an ear canal of a person, comprising: measuring the inner shape of the person's outer ear and ear canal and forming a hard shell (11, 12, 50, 112, 1000), wherein the outer surface of said shell is individually shaped according to said measured inner shape of the person's outer ear and ear canal for optimized fit therein and is adapted to attenuate sound waves arriving at the outer end of the earplug, and wherein tracing means (58) adapted for being detected by an electromagnetic detector are integrated within said shell.

28. A method for manufacturing a passive hearing protection earplug for being worn at least partly within an ear canal of a person, comprising: measuring the inner shape of the person's outer ear and ear canal, forming a hard shell (11, 12, 50, 112, 1000), and fixing tracing means (25, 30, 35, 42, 44, 46, 54, 60, 118, 122, 128, 140, 1002, 1004, 1006, 1010, 1013, 1014) at said shell, wherein the outer surface of said shell is individually shaped according to said measured inner shape of the person's outer ear and ear canal for optimized fit therein and is adapted to attenuate sound waves arriving at the outer end of the earplug.

29. The method of claim 28, wherein, when forming said shell (50, 1000), a receptacle portion (52, 1012) for inserting a metal part (54, 1013) as said tracing means in said shell is pre-shaped in said shell.

30. The method of claim 29, wherein said metal part (54, 1013) is pressure mounted in said receptacle portion (52, 1012).

31. The method of claim 27, wherein when forming said shell (50), metal particles (58) are dispersed within the shell material (56) for forming said tracing means.

32. The method of one of claims 27 to 31, wherein said shell (11, 12, 50, 112, 1000) is built up by an additive process.

33. The method of claim 32, wherein said shell (11, 12, 50, 112, 1000) is formed by layer-by-layer laser sintering of a powder material (56).

34. The method of claim 33, wherein said shell powder material (56) is mixed with metal particles (58) before forming said shell (50).

35. Method for detecting, when lost, the position of a hearing protection earplug according to one of claims 1 to 26, wherein an electromagnetic detector is used for detecting the position of said tracing means (25, 30, 35, 42, 44, 46, 54, 58, 60, 90, 118, 122, 128, 140, 1002, 1004, 1006, 1010, 1013, 1014).

36. Method for detecting, when lost, the position of an active hearing protection earplug for being worn at least partly within the ear canal of a person, said earplug comprising a hard shell (2000) having an outer surface individually shaped according to the measured inner shape of the person's outer ear and ear canal and being adapted to attenuate sound waves arriving at the outer end of the earplug, and further comprising a plurality of electronic components (2002, 2010) capable of a providing a controlled sound transmission through the earplug by converting sound to an audio signal which is processed and reconverted to sound, wherein an electromagnetic detector is used for detecting the position of at least one (2002, 2010) of said electrical components.
